# EUROPEAN PATENT APPLICATION

(11) **EP 4 239 639 A1**
(43) Date of publication of application: **06.09.2023**
(21) Application number: 22207165.6
(22) Date of filing: 14.11.2022
(51) Int. Cl.: G16C 10/00

(54) **SIMULATION PROGRAM, SIMULATION METHOD, AND SIMULATION DEVICE**

(30) Priority: 02.03.2022 JP 2022031831
(71) Applicant: FUJITSU LIMITED, Kawasaki-shi, Kanagawa 211-8588 (JP)
(72) Inventor: Tamiya, Yutaka, Kawasaki-shi, Kanagawa, 211-8588 (JP)
(74) Representative: Hoffmann Eitle

(57) **Abstract**

A simulation program that causes a computer to execute a process includes acquiring coordinates of particles disposed in a virtual space, moving, in a first coordinate axis direction among coordinate axes that define the coordinates on the virtual space, a window with a width of a cutoff radius used for cutoff of calculation of an interaction between two particles, determining whether first particles that enter the window by the moving and second particles that exist in the window are within the cutoff radius and, when the first particles and the second particles exist, generating a particle pair, and iterating the moving of the window and the generating of the particle pair.

## Description

### [Technical Field]

The embodiments discussed herein are related to a simulation technology.

### [Background Art]

In the density functional theory (DFT) used in quantum chemistry simulations, the calculation of interactions of electron pairs, that is, the amount of calculations of two-electron integrals has been given as one of the bottlenecks.

From the aspect of speeding up such calculation of the interactions, a threshold for the distance at which the calculation of the interactions is terminated may be set as a cutoff radius rc. In other words, from the aspect that the interaction becomes weaker as the distance between the electron pair increases, the amount of calculations of the interactions is reduced by calculating the interaction while narrowing down to electron pairs within the cutoff radius rc.

However, even if the above cutoff radius is introduced, it is still difficult to speed up the calculation of the interactions because 90% or more of the execution time by a general-purpose processor is spent on the two-electron integrals.

For example, a framework of a quantum chemical simulator has been proposed that aims to speed up the calculation of interactions by distributed parallelization. In this quantum chemical simulator, the electrons in the list of electrons handled in the simulation are equally divided from the top to the bottom, and the equally divided electrons are assigned to multiple computer nodes. Each computer node searches, for each assigned electron, the vicinity of that electron and extracts the electrons that make up the pair.

### [Citation List]

### [Patent Literature]

Patent document 1: Japanese Laid-open Patent Publication No. 2013-211006
Patent document 2: Japanese Laid-open Patent Publication No. H8-285757
Patent document 3: U.S. Patent Application Publication No. 2017/0193251,
Patent document 4: U.S. Patent No. 4,908,781.

### [Summary of invention]

### [Technical Problem]

However, the quantum chemical simulator described above may be unable to reduce the number of cutoff determinations because the cutoff determination is carried out for each combination of all electron pairs to determine whether the distance between the electron pair is within the cutoff radius. Note that, here, the example of the quantum chemistry simulation is given just as one aspect; however, similar issues can arise in general simulations of the dynamics of multi-particle systems, such as molecular dynamics.

Accordingly, it is an object in one aspect of an embodiment of the present invention to provide a simulation program, a simulation method, and a simulation device that can reduce the number of cutoff determinations.

### [Solution to Problem]

An aspect of a simulation program that causes a computer to execute a process includes: acquiring coordinates of particles disposed in a virtual space; moving, in a first coordinate axis direction among coordinate axes that define the coordinates on the virtual space, a window with a width of a cutoff radius used for cutoff of calculation of an interaction between two particles; determining whether first particles that enter the window by the moving and second particles that exist in the window are within the cutoff radius and, when the first particles and the second particles exist, generating a particle pair; and iterating the moving of the window and the generating of the particle pair.

### [Advantageous Effects of Invention]

The number of cutoff determinations can be reduced.

### [Brief Description of Drawings]

FIG. 1 is a block diagram illustrating a function structure example of a server device;
FIG. 2 is a block diagram illustrating a function structure example of a pair generation unit;
FIG. 3 is a schematic diagram illustrating an example of a cutoff area;
FIG. 4 is a schematic diagram (1) illustrating an example of transition of a window;
FIG. 5 is a schematic diagram (2) illustrating an example of the transition of the window;
FIG. 6 is a schematic diagram (3) illustrating an example of the transition of the window;
FIG. 7 is a schematic diagram (4) illustrating an example of the transition of the window;
FIG. 8 is a schematic diagram (5) illustrating an example of the transition of the window;
FIG. 9 is a flowchart expressing a procedure of a pair generating process; and
FIG. 10 is a diagram illustrating a hardware structure example.

### [Embodiments for Carrying Out the Invention]

Preferred embodiments will be explained with reference to accompanying drawings. Each example merely describes one example or aspect, and the range of numerals and functions, usage scenarios, and the like are not limited by such examples. The examples can be combined as appropriate to the extent that the processing contents are not contradictory.

### First Embodiment

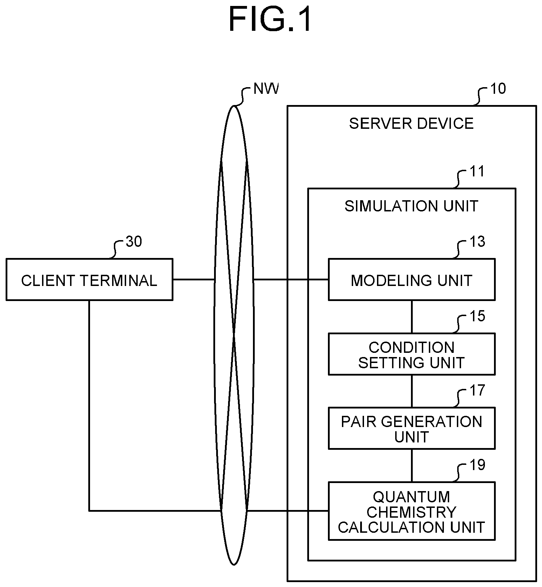

FIG. 1 is a block diagram illustrating a function structure example of a server device 10. The server device 10 illustrated in FIG. 1 provides simulation functions for multi-particle systems such as quantum chemistry and molecular dynamics.

The server device 10 is an example of a computer that provides the simulation functions described above. As an embodiment, the server device 10 can provide the simulation functions described above by executing simulation programs that implement the simulation functions described above. For example, the server device 10 can be realized as a server that provides the above simulation functions on-premise. In addition, the server device 10 can be realized as a Platform as a Service (PaaS) or Software as a Service (SaaS) type application. This also allows the server device 10 to provide the above simulation functions as a cloud service for high performance computing (HPC).

As illustrated in FIG. 1, the server device 10 can be communicatively connected to a client terminal 30 via a network NW. For example, the network NW may be any type of communication networks, wired or wireless, such as the Internet or a local area network (LAN). Although FIG. 1 illustrates an example in which one client terminal 30 is connected per server device 10, any number of client terminals 30 may be connected.

The client terminal 30 corresponds to an example of a computer that receives the simulation functions described above. For example, the client terminal 30 may be realized by a desktop or laptop personal computer. This is just an example; the client terminal 30 may be any computer, such as a portable terminal device or wearable terminal.

Although FIG. 1 illustrates an example where the above simulation functions are provided by a client-server system, this is just an example, and the above simulation functions may be provided on a stand-alone basis.

Next, the function structure example of the server device 10 in this example is described. FIG. 1 schematically illustrates blocks related to the simulation functions of the server device 10. As illustrated in FIG. 1, the server device 10 includes a simulation unit 11. FIG. 1 merely illustrates the extracted functional units related to the above simulation functions, and the functional units other than those illustrated in the drawing, such as a communication control unit and a storage unit not illustrated in the drawing, may be provided in the server device 10.

The simulation unit 11 is a processing unit that provides the simulation functions described above. As a mere example, the simulation unit can be realized by a hardware processor. For example, the processor, by executing the above simulation program, causes the process corresponding to the above simulation unit 11 to be loaded on a memory, which is not illustrated. This allows the above simulation unit 11 to be virtually realized as a process. In addition, the simulation unit 11 or a part of the simulation unit 11 may be implemented by hardwired logic.

As illustrated in FIG. 1, the simulation unit 11 includes a modeling unit 13, a condition setting unit 15, a pair generation unit 17, and a quantum chemistry calculation unit 19. These functional units including the modeling unit 13, the condition setting unit 15, the pair generation unit 17, and the quantum chemistry calculation unit 19 do not have to be provided in a packaged state as the simulation functions described above. For example, some of the above functional units may be referred to as libraries or provided as modules.

The modeling unit 13 is a processing unit that performs modeling of particles. As just one example of such an environment for modeling particles, the modeling unit 13 can provide a graphical user interface (GUI) environment that enables operations related to display of two- or three-dimensional view of molecules and drawing of chemical structures. In addition, multi-particle systems can be modeled by searching a database of molecules and molecular structures of the type appropriate to the usage scenarios, such as pharmaceuticals, drug discovery, or materials. Such modeling of the multi-particle system yields a list of particles with coordinates as a particle list.

The condition setting unit 15 is a processing unit that sets conditions related to the quantum chemistry calculations. As just one example, the condition setting unit 15 can receive settings for conditions related to an experimental environment, such as temperature and pressure. In addition, the condition setting unit 15 can also receive the setting of the potential function used in the density functional theory.

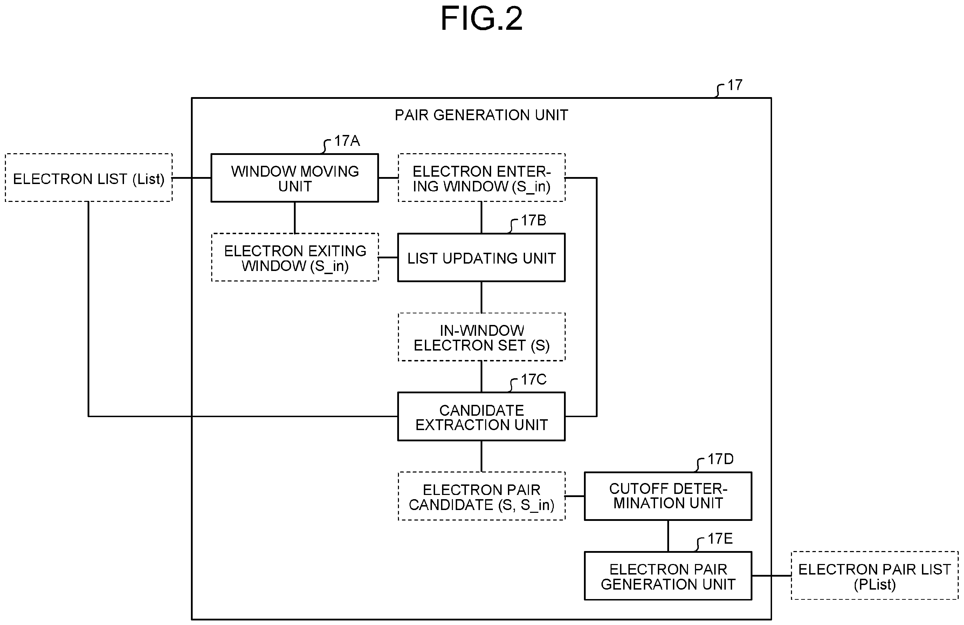

The pair generation unit 17 is a processing unit that generates pairs of particles used in quantum chemistry calculations to calculate the interaction between two particles. From the aspect of reducing the number of cutoff determinations, the pair generation unit 17 realizes the following pair generating function and the details are described below with reference to FIG. 2 to FIG. 8. In other words, the pair generation unit 17 generates pairs of particles that are within the cutoff radius while excluding, from the list of particles resulting from the modeling, pairs of particles for which the difference in coordinate in any of the x, y, and z directions in the three dimensions is greater than the cutoff radius. With such a pair generating function, a list of particle pairs is obtained as a particle pair list.

The quantum chemistry calculation unit 19 is a processing unit that performs quantum chemistry calculations on the basis of the density functional theory. As just one example, the quantum chemistry calculation unit 19 calculates molecular orbitals, i.e., the electron density distribution and energy of the ground state, by narrowing down to the particle pair generated by the pair generation unit 17 and performing the calculation of the interaction between the two particles. The quantum chemistry calculation unit 19 then performs various simulations of molecular structures, reactivity, physical properties, and the like on the basis of the molecular orbitals. The simulation results thus obtained are output to any output destination, such as the client terminal 30.

Next, the details of the aforementioned pair generating function are described. FIG. 2 is a block diagram illustrating a function structure example of the pair generation unit 17. In FIG. 2, the blocks related to the pair generating function applied in the usage scenarios in which quantum chemistry simulations are performed are schematically illustrated, and the blocks corresponding to the data input/output by the respective units are schematically illustrated with dashed lines. FIG. 2 illustrates the quantum chemistry simulation as just one example of the usage scenarios, but the present invention is not limited to this example. For example, by replacing electrons with ordinary particles, the above pair generating function can be applied to simulations of the dynamics of the multi-particle systems in general, such as molecular dynamics simulations and astronomy simulations dealing with gravity.

The pair generation unit 17 illustrated in FIG. 2 receives the input of an electron list corresponding to one example of the particle list and outputs an electron pair list PList corresponding to one example of the particle pair list. As illustrated in the drawing, the pair generation unit 17 includes a window moving unit 17A, a list updating unit 17B, a candidate extraction unit 17C, a cutoff determination unit 17D, and an electron pair generation unit 17E.

The window moving unit 17A is a processing unit that moves a window W to search for an electron in a virtualized three-dimensional space (hereinafter described as "virtual space"). The following is an example where the coordinates on the virtual space are defined by the Cartesian coordinate system represented by three coordinate axes: the x-axis, the y-axis, and the z-axis, which are orthogonal to each other. Under such a coordinate system, the window W with a width corresponding to the cutoff radius rc in the z direction is used as an example and a description is continued below.

As an embodiment, the window moving unit 17A acquires the electron list List obtained from the modeling by the modeling unit 13. For example, the electron list List refers to data that lists the electrons present in the virtual space and may include the coordinates of each electron. The term "coordinates of an electron" here refers to the position of the orbital center of the electron, i.e., the center of the atom.

After such an electron list List is acquired, the window moving unit 17A sorts the electrons in the electron list List on the basis of the coordinate values corresponding to one of the three coordinate axes. The coordinate values used for sorting and the type of sorting correspond to the direction in which the window W moves. For example, if the window W is moved in the positive direction of the z-axis, the electrons in the electron list List are sorted in ascending order of the z-coordinate.

The window moving unit 17A then takes the initial position of the window W as the minimum value of the z-coordinate and slides the window W by a specific shift amount Δz in the positive direction of the z-axis from that position. Each time the window W is slid in this manner, the window moving unit 17A calculates an electron S in entering the window W and an electron S_out exiting the window W. After that, the window moving unit 17A iteratively moves the window W until the end condition for moving the window W is satisfied, for example, until both the electron list List and an in-window electron set S become an empty set ϕ.

The list updating unit 17B is a processing unit that updates the electron list List and the in-window electron set S. For example, the in-window electron set S refers to the data listing the set of electrons in the window W at the current location.

As an embodiment, the list updating unit 17B updates the electron list List and the in-window electron set S on the basis of the calculation results of the electron S_in entering the window W and the electron S out exiting the window W by the window moving unit 17A.

As one aspect, the list updating unit 17B performs an update of deleting the electron S_in entering the window W from the electrons in the electron list List. As another aspect, the list updating unit 17B performs an update of adding the electron S_in entering the window W to the in-window electron set S, or deleting the electron S_out exiting the window W from the in-window electron set S.

The candidate extraction unit 17C is a processing unit that extracts the electron pair whose cutoff areas overlap each other as a candidate electron pair. The "cutoff area" here is set based on the cutoff radius rc for each electron in the electron list List.

As just one example, the candidate extraction unit 17C sets the coordinates of the electron as a start point for each electron in the electron list List, and sets the coordinates obtained by adding the cutoff radius rc to the x-coordinate, y-coordinate, and z-coordinate of the start point as an end point. This yields a cubic area containing diagonally the line segments including the start point and the end point as the cutoff area.

Under such a cutoff area setting, the candidate extraction unit 17C determines whether the cutoff areas overlap between the in-window electron set S and the electron S_in entering the window W.

Here, when the cutoff areas overlap each other, the distance of the electron pair with the overlapping cutoff areas in all the x-, y- and z-directions can be confirmed to be less than or equal to the cutoff radius rc. In this case, the candidate extraction unit 17C extracts the electron pair with the overlapping cutoff areas as the electron pair candidate (S, S in).

The cutoff determination unit 17D is a processing unit that performs the cutoff determination described above. As one embodiment, the cutoff determination unit 17D calculates the distance of the electron pair candidate (S, S_in) extracted by the candidate extraction unit 17C. The cutoff determination unit 17D then determines whether the distance of the electron pair candidate (S, S_in) is less than or equal to the cutoff radius rc.

The electron pair generation unit 17E is a processing unit that generates electron pairs. As one embodiment, from among the electron pair candidates (S, S_in) extracted by the candidate extraction unit 17C, the electron pair generation unit 17E generates as an electron pair S', the electron pair candidate (S, S in) for which the cutoff determination unit 17D has determined the distance is less than or equal to the cutoff radius rc. The electron pair generation unit 17E then adds the electron pair S' to the electron pair list PList containing a list of electron pairs.

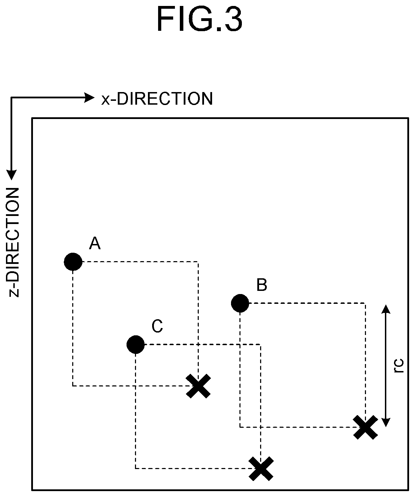

Next, a specific example of a method of generating the electron pairs is described with reference to FIG. 3 to FIG. 8. An example of the electrons included in the electron list List and the cutoff areas set for those electrons is given below. While the movement of the window W is given chronologically as an example, the overlap determination of the cutoff areas, the cutoff determination, and the generation of electron pairs that are performed at the respective stages are described.

FIG. 3 is a schematic diagram illustrating an example of the cutoff area. In FIG. 3, three electrons, an electron A, an electron B, and an electron C, are illustrated as examples of the electrons in the electron list List, and the coordinates of the three electrons are illustrated with circular marks. In addition, FIG. 3 illustrates the outline of the cutoff area set for each of the three electrons, the electron A, the electron B, and the electron C, with dashed lines. For convenience of the description, the two-dimensional plane in the x-direction and the z-direction is used as an example in FIG. 3, but the process similar to that in the x-direction may be performed in the y-direction also in a three-dimensional space including the y-direction and it is obvious that extension to the three-dimensional space is possible.

As illustrated in FIG. 3, the coordinates of the electron A, the electron B, and the electron C are set as the start points, and the coordinates obtained by adding the cutoff radius rc to the x-coordinates and the z-coordinates of the start points, or X-marks, are set as the end points. Accordingly, a square area containing the start point and the end point diagonally is obtained as the cutoff area for each of the electron A, the electron B, and the electron C.

The cutoff area is set according to a common rule among all electrons, for example, the addition of the cutoff radius rc to each coordinate value of the electron. Thus, the relative position of the cutoff area between all electrons can be regarded as that in the case where the cutoff area is set with the coordinates of the electron as a center.

Furthermore, by setting a rectangular cutoff area, the overlap determination between the cutoff areas can be simplified. For example, in terms of a two-dimensional plane, the overlap of the cutoff areas can be determined by the overlap of rectangular cutoff areas, which can reduce the amount of calculations compared to the case of determining the overlap of circular cutoff areas centered on the coordinates of the electron. Even in the three-dimensional space, the overlap of the cutoff areas can be determined by the overlap of cubic cutoff areas, which can reduce the amount of calculations compared to the case of determining the overlap of spherical cutoff areas centered on the coordinates of the electron.

Under the setting of the cutoff areas for the electron A, the electron B and the electron C, the window W with a width corresponding to the cutoff radius rc in the z direction starts to move.

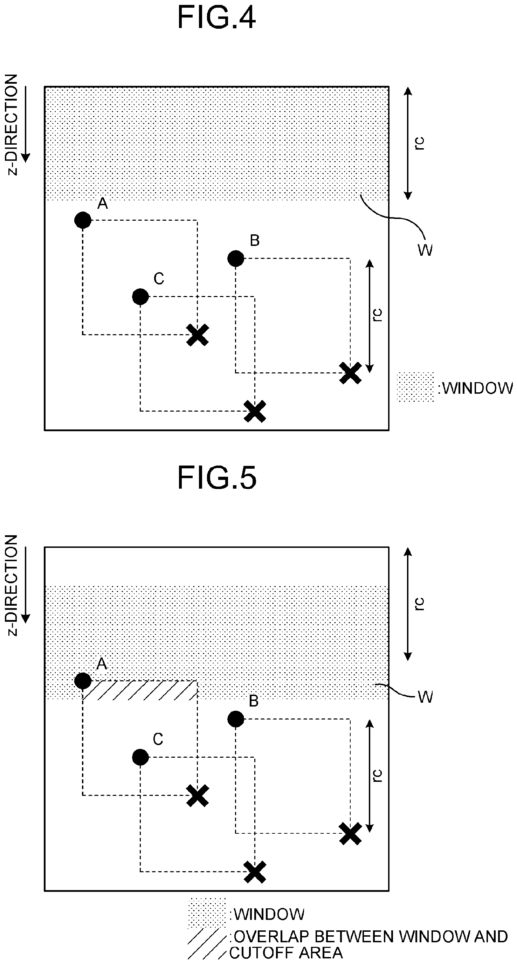

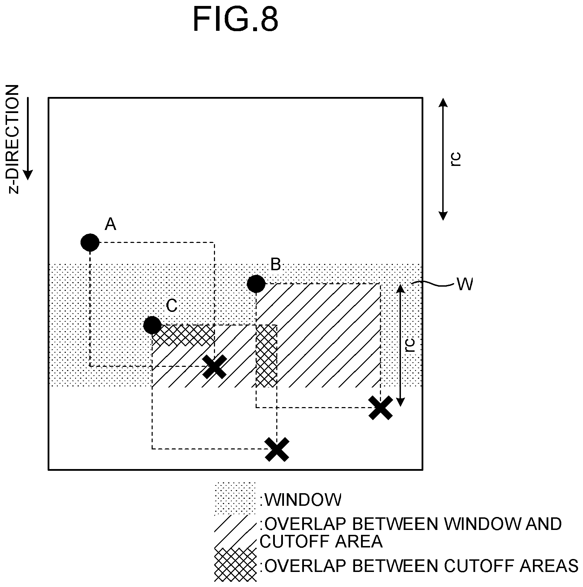

FIG. 4 and FIG. 8 are schematic diagrams (1) to (5) illustrating examples of the transition of the window W. While FIG. 4 illustrates the initial position of the window W, FIG. 5 to FIG. 8 illustrate how the window W is moved chronologically from its initial position toward the positive direction of the z-axis in the order of the figure number of FIG. 5 to FIG. 8.

As illustrated in FIG. 4, when the window W is in the initial position, the window W does not contain any of the electron A, the electron B, and the electron C. Therefore, the processes by the list updating unit 17B, the candidate extraction unit 17C, the cutoff determination unit 17D, and the electron pair generation unit 17E are not performed at the stage when the window W is in the initial position. Each list at the initial position of the window W will be as follows.
Electron list List = {A, B, C}
In-window electron set S = {ϕ}
Electron pair list PList = {ϕ}

After that, the window moving unit 17A moves the window W from the initial position of the window W illustrated in FIG. 4 to the position of the window W illustrated in FIG. 5. At this point, the start point of the electron A enters the window W as illustrated in FIG. 5, and thus, the window moving unit 17A identifies the electron A as the electron S_in entering the window W. Therefore, the list updating unit 17B performs updates of deleting the electron A from the electron list List and adding the electron A to the in-window electron set S. At this stage, since nothing but the electron A is included in the window W, the processes by the candidate extraction unit 17C, the cutoff determination unit 17D, and the electron pair generation unit 17E are not performed.
Electron entering the window W S in = {A}
Electron list List = {B, C}
In-window electron set S = {A}
Electron pair list PList = {ϕ}

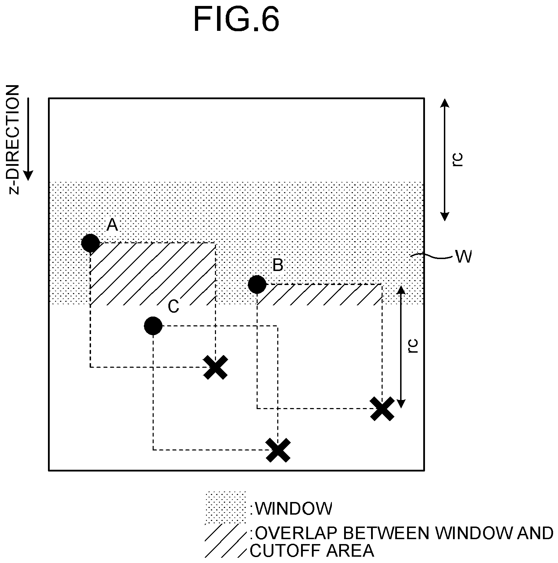

The window moving unit 17A then moves the window W from the position of the window W illustrated in FIG. 5 to the position of the window W illustrated in FIG. 6. At this point, the start point of the electron B enters the window W as illustrated in FIG. 6, and thus, the window moving unit 17A identifies the electron B as the electron S in entering the window W. Therefore, the list updating unit 17B performs updates of deleting the electron B from the electron list List and adding the electron B to the in-window electron set S. Furthermore, the candidate extraction unit 17C determines whether the cutoff areas overlap between the electron A corresponding to the in-window electron set S and the electron B corresponding to the electron S_in entering the window W. In this case, the cutoff area of the electron A and the cutoff area of the electron B do not overlap; therefore, the pair of the electron A and the electron B is not extracted as the electron pair candidate, and the processes by the cutoff determination unit 17D and the electron pair generation unit 17E are not performed.
Electron entering the window W S in = {B}
Electron list List = {C}
In-window electron set S = {A, B}
Electron pair list PList = {ϕ}

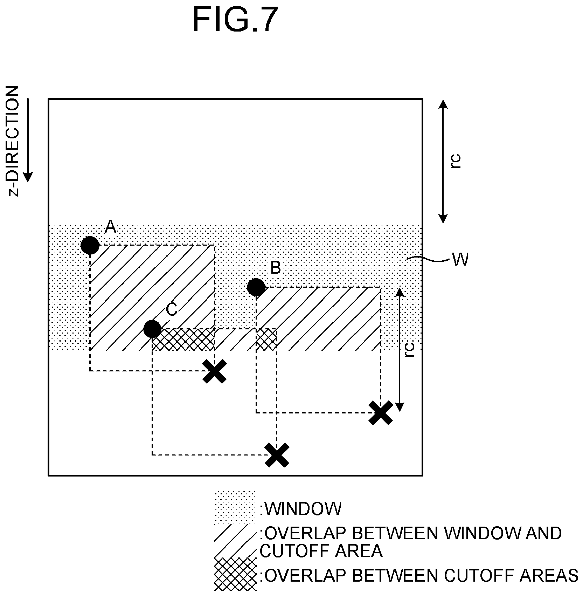

Furthermore, the window moving unit 17A moves the window W from the position of the window W illustrated in FIG. 6 to the position of the window W illustrated in FIG. 7. At this point, the start point of the electron C enters the window W as illustrated in FIG. 7, and thus, the window moving unit 17A identifies the electron C as the electron S_in entering the window W. Therefore, the list updating unit 17B performs updates of deleting the electron C from the electron list List and adding the electron C to the in-window electron set S.

Furthermore, the candidate extraction unit 17C determines whether the cutoff areas overlap between the electron A and the electron B corresponding to the in-window electron set S and the electron C corresponding to the electron S in entering the window W. In this case, as indicated by hatching in FIG. 7, the cutoff areas overlap between the electron A and the electron C and the cutoff areas overlap between the electron B and the electron C.

By the overlap of the cutoff areas, the x-directional distance between the electron A and the electron C can be confirmed to be within the cutoff radius rc, and also the x-directional distance between the electron B and the electron C can be confirmed to be within the cutoff radius rc. Therefore, the electron A and the electron C, and the electron B and the electron C are extracted as the electron pair candidates (S, S_in) by the candidate extraction unit 17C.

Here, the cutoff determination unit 17D determines whether the distance of the electron pair candidate of the electron A and the electron C is within the cutoff radius rc, and whether the distance of the electron pair candidate of the electron B and the electron C is within the cutoff radius rc. At this time, in this example, the distance between the electron pair candidate of the electron A and the electron C is within the cutoff radius rc, and the distance between the electron pair candidate of the electron B and the electron C is within the cutoff radius rc. In this case, the electron pair generation unit 17E performs the generation of an electron pair S'1 of the electron A and the electron C, "S'1 = MakePair (electron A, electron C)", and the generation of an electron pair S'2 of the electron B and the electron C, "S'2 = MakePair (electron B, electron C)". Then, the electron pair generation unit 17E performs the update of adding the electron pair S'1 and the electron pair S'2 to the electron pair list PList, "PList = ϕ + electron pair S'1 + electron pair S'2".
Electron entering window W S in = {C}
Electron list List = {ϕ}
In-window electron set S = {A, B, C}
Electron pair list PList = {S'1, S'2}

After that, the window moving unit 17A moves the window W from the position of the window W illustrated in FIG. 7 to the position of the window W illustrated in FIG. 8. At this point, the end point of the electron A exits the window W as illustrated in FIG. 8, and thus, the window moving unit 17A identifies the electron A as the electron S_out exiting the window W. Therefore, the list updating unit 17B performs the update of deleting the electron A from the in-window electron set S. In this case, the electron S in entering the window W does not exist, so that the processes by the candidate extraction unit 17C, the cutoff determination unit 17D, and the electron pair generation unit 17E are not performed.
Electron exiting window W S out = {A}
Electron list List = {ϕ}
In-window electron set S = {B, C}
Electron pair list PList = {S'1, S'2}

Then, although the illustration is omitted, when the window W is moved by the window moving unit 17A to the position beyond the end point of the electron B, the window moving unit 17A identifies the electron B as the electron S_out exiting the window W. Then, the list updating unit 17B performs the update of deleting the electron B from the in-window electron set S. In this case, the electron S_in entering the window W does not exist, so that the processes by the candidate extraction unit 17C, the cutoff determination unit 17D, and the electron pair generation unit 17E are not performed.
Electron exiting window W S out = {B}
Electron list List = {ϕ}
In-window electron set S = {C}
Electron pair list PList = {S'1, S'2}

Furthermore, when the window moving unit 17A has moved the window W to the position beyond the end point of the electron C, the window moving unit 17A identifies the electron C as the electron S_out exiting the window W. Then, the list updating unit 17B performs the update of deleting the electron C from the in-window electron set S. In this case, the electron S in entering the window W does not exist, so that the processes by the candidate extraction unit 17C, the cutoff determination unit 17D, and the electron pair generation unit 17E are not performed.
Electron exiting window W S out = {C}
Electron list List = {ϕ}
In-window electron set S = {ϕ}
Electron pair list PList = {S'1, S'2}

In this manner, when the electron list List and the in-window electron set S become the empty set ϕ, the generation of electron pairs is terminated. As a result, the electron pair S'1 and the electron pair S'2 are generated as the electron pairs. To generate these electron pairs S'1 and S'2, according to the electron pair generating function in this example, two cutoff determinations are performed, that is, the cutoff determination between the electron A and the electron C and the cutoff determination between the electron B and the electron C. On the other hand, the cutoff area overlap determination between the electron A and the electron B avoids the cutoff determination between the electron A and the electron B.

According to the conventional technology described above, if three electrons, the electron A, the electron B, and the electron C, are included in the electron list, the cutoff determination is performed three times corresponding to all combinations ₃C₂, so that electron pairs similar to those by the electron pair generating function in this example can be given.

Therefore, according to the electron pair generating function in this example, the cutoff determination between the electron A and the electron B can be reduced compared to the conventional technology described above.

Here, in FIG. 3 to FIG. 8 illustrating just one example, the difference in the z-coordinate is within the cutoff radius rc for all combinations of the electron A, the electron B, and the electron C. However, there may be combinations of electrons in the electron list List where the difference in the z-coordinate exceeds the cutoff radius rc. If there is a combination of electrons for which the difference in the z-coordinate exceeds the cutoff radius rc in this manner, such a combination is excluded from the electron pair candidates and the combinations of the in-window electron set S and electrons entering the window W S_in corresponding to the target of the electron pair. Therefore, it is obvious that the electron pair generating function in this example can reduce the cutoff area overlap determination and the cutoff determination regarding the combinations of the electrons whose difference in the z-coordinate exceeds the cutoff radius rc.

Furthermore, by managing the coordinates of the electron set by the window, the electron pair generating function in this example can limit the electron pair candidates within the cutoff radius to those within the window. For example, when the number of electrons is N, the calculation time is O(N log2 N). This is more efficient than the above conventional technique with the calculation time O(N^2) for all electron pair candidates.

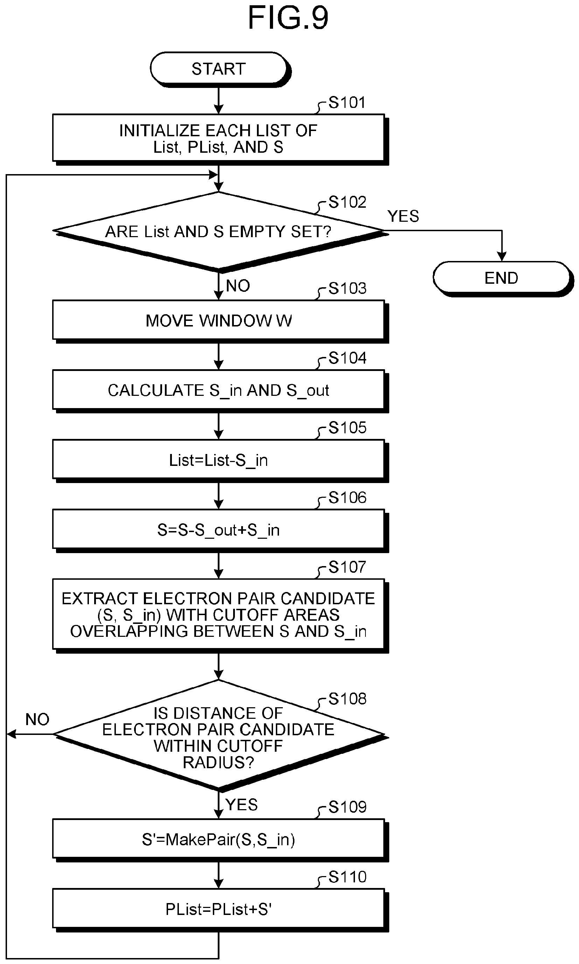

Next, the procedure of the process of the server device 10 in this example will be described. FIG. 9 is a flowchart of the procedure of the pair generating process. This process can be started when, for example, the electron list List is acquired.

As illustrated in FIG. 9, the electron list List, the in-window electron set S, and the electron pair list PList are initialized (step S101). For example, the electron list List is sorted in the ascending order of the z-coordinate, and the in-window electron set S and the electron pair list PList are set to the empty set ϕ.

Then, the process from step S103 to step S110 below is iterated until both the electron list List and the in-window electron set S become the empty set ϕ (No at step S102).

That is, the window moving unit 17A takes the initial position of a bottom surface of the window W as the minimum value of the z-coordinate, and moves the window W in the positive direction of the z-axis from that position by a specific shift amount Δz (step S103). The window moving unit 17A then calculates the electron S_in entering the window W and the electron S out exiting the window W (step S104).

Then, the list updating unit 17B performs the update of deleting the electron S_in entering the window W among the electrons in the electron list List (step S105). In addition, the list updating unit 17B performs the updates of adding the electron S in entering the window W to the in-window electron set S, deleting the electron S_out exiting the window W from the in-window electron set S, or both adding and deleting (step S106).

The candidate extraction unit 17C then extracts the electron pair with the overlapping cutoff areas between the in-window electron set S and the electron S_in entering the window W, as the electron pair candidate (S, S_in) (step S107).

Then, the cutoff determination unit 17D determines whether the distance of the electron pair candidate (S, S_in) extracted at step S107 is less than or equal to the cutoff radius rc (step S108) .

If the distance of the electron pair candidate (S, S_in) is less than or equal to the cutoff radius rc (Yes at step S108), the electron pair generation unit 17E generates the electron pair candidate (S, S_in) as the electron pair S' (step S109). Then, the electron pair generation unit 17E adds the electron pair S' generated at step S109 to the electron pair list PList (step S110) and the process advances to step S102.

On the other hand, if the distance of the electron pair candidate (S, S_in) is not less than or equal to the cutoff radius rc (No at step S108), the processes at steps S109 and S110 are skipped and the process advances to step S102.

Then, if both the electron list List and the in-window electron set S become the empty set ϕ (Yes at step S102), the process is terminated.

As mentioned above, the server device 10 in this example iteratively moves the window with a width of the cutoff radius rc of the calculation of the interaction between two particles in one axial direction, and generates pairs within rc between the electrons entering the window and the electrons in the window. Therefore, with the server device 10 in this example, the cutoff determination between the electrons whose coordinate difference exceeds rc can be reduced. Furthermore, the effect of reducing the number of cutoff determinations is more enhanced in applications to simulations of quantum dynamical systems in which the coordinates of the electrons are updated as the simulation time progresses.

Furthermore, the server device 10 in this example extracts, as the electron pair candidate, the electron pair with the overlapping cutoff areas set based on the cutoff radius rc for each electron in the electron list. Therefore, with the server device 10 in this example, the cutoff determinations between the electrons whose coordinate difference is within rc can be reduced according to the results of the overlapping determination about the cutoff areas with a smaller amount of calculations than the cutoff determination.

### Second Embodiment

The example of the disclosed device has been described so far, but the present invention may be implemented in a variety of different forms in addition to the example described above. Therefore, other examples included in the present invention are described below.

### Extension Example 1

For example, the z-coordinate of the start point of the electron and the z-coordinate of the end point of the electron may be registered in the electron list List. Such an electron list List can be sorted in the ascending order of the z-coordinate and then used to generate the electron pairs. In this case, the window moving unit 17A can determine the amount of movement of the window W on the basis of the coordinates of the electrons obtained from the electron list List. For example, if the z-coordinate of the electron acquired from the electron list List is the z-coordinate of the start point, the window moving unit 17A can move the window W to the z-coordinate of that start point and identify the electron acquired from the electron list List as the electron S_in entering the window W. On the other hand, if the z-coordinate of the electron acquired from the electron list List is the z-coordinate of the end point, the window moving unit 17A can move the window W to the z-coordinate of that end point and identify the electron acquired from the electron list List as the electron S_out exiting the window W. Such an extension enables the management of electron entry and exit to and from the window with a minimum number of moves.

### Extension Example 2

As an extension of the in-window electron set S, two lists can be kept: XList sorted by the x-coordinate and YList sorted by the y-coordinate. Under the circumstances where these two lists, XList and YList, are kept, when the electron S_out exiting the window W is obtained, the x-coordinate of the electron S_out can be retrieved from XList and the y-coordinate of the electron S_out can be retrieved from YList. In this case, when the number of electrons held in the in-window electron set S is NS, the retrieval time is O(log2 NS). Thus, the retrieval is more efficient than when the in-window electron set S is a single list. Similarly, when the electron S_in entering the window W is obtained, the sort position where the x-coordinate of the electron S_in is registered in XList and the sort position where the y-coordinate of the electron S_in is registered in YList can be calculated by O(log2 NS). The candidate extraction unit 17C can also calculate the retrieval for the electrons in the range of ± cutoff radius from the x-coordinate and the y-coordinate of the electron S_in entering the window with O(log2 NS) using XList and YList. Therefore, the electron pair generation can also be performed efficiently.

### Distribution and Integration

In addition, each component of each device in the drawings does not necessarily have to be physically configured as illustrated in the drawings. In other words, the specific form of dispersion and integration of each device is not limited to that illustrated in the drawings, but can be configured by functionally or physically dispersing or integrating the entirety or part of it in arbitrary units according to various loads and usage conditions. For example, the window moving unit 17A, the list updating unit 17B, the candidate extraction unit 17C, the cutoff determination unit 17D, or the electron pair generation unit 17E may be connected via a network as the external device of the pair generation unit 17. The window moving unit 17A, the list updating unit 17B, the candidate extraction unit 17C, the cutoff determination unit 17D, or the electron pair generation unit 17E may be provided in different devices respectively and connected to the network so as to cooperate with each other to realize the functions of the pair generation unit 17 described above.

### Hardware Structure

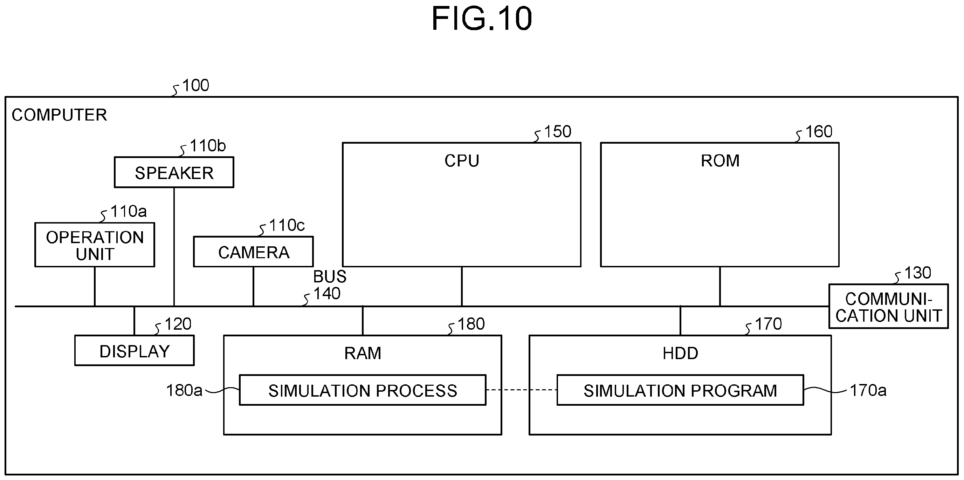

Various processes described in the above examples can be realized when a computer such as a personal computer or workstation executes computer programs prepared in advance. Therefore, an example of a computer that executes a simulation program with the functions similar to those in Example 1 and Example 2 is described below with reference to FIG. 10.

FIG. 10 is a diagram illustrating a hardware structure example. As illustrated in FIG. 10, a computer 100 includes an operation unit 110a, a speaker 110b, a camera 110c, a display 120, and a communication unit 130. In addition, this computer 100 includes a central processing unit (CPU) 150, a read only memory (ROM) 160, a hard disk drive (HDD) 170, and a random access memory (RAM) 180. These components 110 to 180 are connected via a bus 140. Although FIG. 10 illustrates the CPU as an example of a processor, this is just an example, and the processor may be a graphics processing unit (GPU) or general-purpose computing on GPU (GPGPU).

As illustrated in FIG. 10, the HDD 170 stores therein a simulation program 170a that performs the function similar to that of the pair generation unit 17 described above in Example 1. This simulation program 170a may be integrated or separated, in the same manner as each component of the pair generation unit illustrated in FIG. 2. In other words, the HDD 170 does not necessarily have to store therein all the data described above in Example 1, and it suffices if the data used for the process is stored in the HDD 170.

Under this environment, the CPU 150 reads out the simulation program 170a from the HDD 170 and then loads this simulation program 170a on the RAM 180. As a result, the simulation program 170a functions as a simulation process 180a as illustrated in FIG. 10. This simulation process 180a loads various kinds of data read out from the HDD 170 on the area allocated to the simulation process 180a among the storage areas in the RAM 180, and executes various processes using the various kinds of data loaded. For example, an example of the process to be performed by the simulation process 180a may include the process illustrated in FIG. 9. The CPU 150 does not necessarily have to operate all the processing units illustrated in Example 1 above, and the processing unit corresponding to the process to be executed may be virtually realized.

The above simulation program 170a does not necessarily have to be stored in the HDD 170 or the ROM 160 from the beginning. For example, the simulation program 170a is stored in a "portable physical medium" such as a flexible disk, so-called FD, a CD-ROM, a DVD disc, a magneto-optical disk, or an IC card that is inserted into the computer 100. The computer 100 may then acquire and execute the simulation program 170a from these portable physical media. In addition, the simulation program 170a is stored in another computer or server device that is connected to the computer 100 via a public line, the Internet, LAN, or WAN. The stored simulation program 170a may be downloaded to the computer 100 and then executed.

## Claims

1. A simulation program that causes a computer to execute a process comprising:
acquiring(S101) coordinates of particles disposed in a virtual space;
moving(S103), in a first coordinate axis direction among coordinate axes that define the coordinates on the virtual space, a window with a width of a cutoff radius used for cutoff of calculation of an interaction between two particles;
determining(S108) whether first particles that enter the window by the moving and second particles that exist in the window are within the cutoff radius and, when the first particles and the second particles exist, generating(S109) a particle pair; and
iterating(S102) the moving of the window and the generating of the particle pair.

2. The simulation program according to claim 1, the process further including:
setting(S107) an area based on the cutoff radius for each particle disposed in the virtual space; and
extracting(S107), from among combinations of the first particles and the second particles, particles with the areas overlapping with each other as a candidate of the particle pair, wherein
the generating includes determining(S108) whether the candidate extracted at the extracting is within the cutoff radius and, when the candidate within the cutoff radius exists, generating(S109) the particle pair.

3. The simulation program according to claim 2, wherein the iterating includes iterating(S102,S103) the moving of the window, based on a start point of the area on the first coordinate axis direction and an end point of the area on the first coordinate axis direction.

4. The simulation program according to claim 1, the process further including searching(S106) for the second particle corresponding to a third particle that exits the window by the moving from each of a first list in which the second particles are sorted by a coordinate value of a second coordinate axis orthogonal to the first coordinate axis and a second list in which the second particles are sorted by a coordinate value of a third coordinate axis orthogonal to the first coordinate axis and the second coordinate axis, and deleting(S106) the second particle from the first list and the second list.

5. The simulation program according to claim 1, the process further including calculating(S104) a sort position at which the first particle that enters the window by the moving is registered as the second particle in a first list in which the second particles are sorted by a coordinate value of a second coordinate axis orthogonal to the first coordinate axis, and calculating(S104) a sort position at which the first particle that enters the window by the moving is registered as the second particle in a second list in which the second particles are sorted by a coordinate value of a third coordinate axis orthogonal to the first coordinate axis and the second coordinate axis.

6. A simulation method comprising:
acquiring(S101) coordinates of particles disposed in a virtual space;
moving(S103), in a first coordinate axis direction among coordinate axes that define the coordinates on the virtual space, a window with a width of a cutoff radius used for cutoff of calculation of an interaction between two particles;
determining(S108) whether first particles that enter the window by the moving and second particles that exist in the window are within the cutoff radius and, when the first particles and the second particles exist, generating a particle pair; and
iterating(S102) the moving of the window and the generating of the particle pair.

7. The simulation method according to claim 6, further including:
setting(S107) an area based on the cutoff radius for each particle disposed in the virtual space; and
extracting(S107), from among combinations of the first particles and the second particles, particles with the areas overlapping with each other as a candidate of the particle pair, wherein
the determining includes determining(S108) whether the candidate extracted at the extracting is within the cutoff radius and, when the candidate within the cutoff radius exists, generating(S109) the particle pair.

8. The simulation method according to claim 7, wherein the iterating includes iterating(S102,S103) the moving of the window, based on a start point of the area on the first coordinate axis direction and an end point of the area on the first coordinate axis direction.

9. The simulation method according to claim 6, further including searching(S106) for the second particle corresponding to a third particle that exits the window by the moving from each of a first list in which the second particles are sorted by a coordinate value of a second coordinate axis orthogonal to the first coordinate axis and a second list in which the second particles are sorted by a coordinate value of a third coordinate axis orthogonal to the first coordinate axis and the second coordinate axis, and deleting(S106) the second particle from the first list and the second list.

10. The simulation method according to claim 6, further including calculating(S104) a sort position at which the first particle that enters the window by the moving is registered as the second particle in a first list in which the second particles are sorted by a coordinate value of a second coordinate axis orthogonal to the first coordinate axis, and calculating(S104) a sort position at which the first particle that enters the window by the moving is registered as the second particle in a second list in which the second particles are sorted by a coordinate value of a third coordinate axis orthogonal to the first coordinate axis and the second coordinate axis.

11. A simulation device(10) comprising:
an acquiring unit(17) configured to acquire coordinates of particles disposed in a virtual space;
a moving unit(17A) configured to move, in a first coordinate axis direction among coordinate axes that define the coordinates on the virtual space, a window with a width of a cutoff radius used for cutoff of calculation of an interaction between two particles;
a determining unit(17D) configured to determine whether first particles that enter the window by the moving and second particles that exist in the window are within the cutoff radius;
a generating unit(17E) configured to generate a particle pair, when the first particles and the second particles exist; and
an iterating unit(17) configured to iterate the moving of the window and the generating of the particle pair.

12. The simulation device(10) according to claim 11, further including:
a setting unit(17C) configured to set an area based on the cutoff radius for each particle disposed in the virtual space; and
an extracting unit(17C) configured to extract, from among combinations of the first particles and the second particles, particles with the areas overlapping with each other as a candidate of the particle pair, wherein
the determining unit(17D) determines whether the candidate extracted at the extracting is within the cutoff radius; and
the generating unit(17E) generates the particle pair, when the candidate within the cutoff radius exists.

13. The simulation device(10) according to claim 12, wherein the iterating unit(17) iterates the moving of the window, based on a start point of the area on the first coordinate axis direction and an end point of the area on the first coordinate axis direction.

14. The simulation device according to claim 11, further including a searching unit(17B) configured to search for the second particle corresponding to a third particle that exits the window by the moving from each of a first list in which the second particles are sorted by a coordinate value of a second coordinate axis orthogonal to the first coordinate axis and a second list in which the second particles are sorted by a coordinate value of a third coordinate axis orthogonal to the first coordinate axis and the second coordinate axis, and delete the second particle from the first list and the second list.

15. The simulation device according to claim 11, further including a calculating unit(17B) configured to calculate a sort position at which the first particle that enters the window by the moving is registered as the second particle in a first list in which the second particles are sorted by a coordinate value of a second coordinate axis orthogonal to the first coordinate axis, and calculate a sort position at which the first particle that enters the window by the moving is registered as the second particle in a second list in which the second particles are sorted by a coordinate value of a third coordinate axis orthogonal to the first coordinate axis and the second coordinate axis.
